# EUROPEAN PATENT APPLICATION

(11) **EP 4 549 562 A1**
(43) Date of publication of application: **07.05.2025**
(21) Application number: 23831290.4
(22) Date of filing: 23.06.2023
(51) Int. Cl.: C12N 9/24, A23C 11/10, A23L 2/38, A23L 11/60, A23L 11/65, A23L 33/20, C12N 9/26, C12P 19/20, C13K 13/00

(54) **ENZYMATIC AGENT FOR LESSENING SUGAR IN VEGETABLE FOOD OR BEVERAGE**

(30) Priority: 28.06.2022 JP 2022103800; 31.08.2022 JP 2022138111
(71) Applicant: Amano Enzyme Inc., Nagoya-shi Aichi 460-8630 (JP)
(72) Inventor: TAKAYAMA Hiroyuki, Kakamigahara-shi, Gifu 509-0109 (JP)
(74) Representative: Mullholland, Lewis Paul
(86) International application number: PCT/JP2023/023291
(87) International publication number: WO 2024/004848

(57) **Abstract**

To provide a technique for reducing a saccharide in a plant-base food or beverage.

The technique provides an enzymatic agent for reducing a saccharide in a plant-base food or beverage, which contains a cyclodextrin-producing enzyme, and a plant-base food or beverage using the enzymatic agent for reducing a saccharide. The technique also provides a method for manufacturing a plant-base food or beverage, which includes a step of allowing a cyclodextrin-producing enzyme to act in a liquefaction process of a plant-base raw material, and a method for reducing a saccharide in a plant-base food or beverage, which includes a step of allowing a cyclodextrin-producing enzyme to act on a plant-base raw material.

## Description

### Technical Field

The present invention relates to an enzymatic agent for reducing a saccharide in a plant-base food or beverage. More specifically, the present invention relates to a technique for reducing a saccharide in a plant-base food or beverage using a cyclodextrin-producing enzyme.

### Background Art

In recent years, a plant-base food or beverage have been attracting attention due to the growing health consciousness. For example, an oat beverage is prepared by suspending oat material in an aqueous medium and decomposing the starch of the oat material using one or more types of amylases. The amylase used in this case is β-amylase or a mixture of α-amylase and β-amylase. The oat beverage prepared by this method gives a moderate sweetness due to the production of maltose by the action of β-amylase (Patent Literature 1).

Here, the enzyme used in the present technique will be described. A cyclodextrin-producing enzyme is an enzyme that acts on starch to produce α-, β-, and γ-cyclodextrins. It is also known that a cyclodextrin-producing enzyme can act on starch to manufacture cyclodextrin without liquefying starch granules (Patent Literature 2).

### Citation List

### Patent Literature

Patent Literature 1: JP-A No. 2018-075029
Patent Literature 2: JP-A No. hei09-262091

### Summary of Invention

### Technical Problem

As mentioned above, a plant-base food or beverage have been attracting attention in recent years due to the growing health consciousness, but for example, a plant-base food or beverage such as oat beverages obtained by treating with β-amylase in addition to α-amylase using the technique shown in Patent Literature 1 contain a large amount of a saccharide typically represented by glucose and maltose. Therefore, the demand for reducing the saccharide content has not been met.

Therefore, the main objective of the present technique is to provide a technique for reducing a saccharide in a plant-base food or beverage.

### Solution to Problem

As a result of intensive research into techniques for reducing a saccharide in a plant-base food or beverage, the inventors of the present application discovered that a saccharide in a plant-base food or beverage can be reduced by allowing a cyclodextrin-producing enzyme to act on a plant-base raw material of the plant-base food or beverage, and thus completed the present technique.

That is, the present technique first provides an enzymatic agent for reducing a saccharide in a plant-base food or beverage, which contains a cyclodextrin-producing enzyme.

The enzymatic agent for reducing a saccharide according to the present technique may also contain a maltotriose-producing enzyme.

The present technique also provides a plant-base food or beverage that uses the enzymatic agent for reducing a saccharide of the present technique.

The plant-base food or beverage according to the present technique may be oat milk.

Next, the present technique provides a method for manufacturing a plant-base food or beverage, which includes a step of allowing a cyclodextrin-producing enzyme to act in a liquefaction process of a plant-base raw material.

The method for manufacturing a plant-base food or beverage according to the present technique may also include a step of allowing a maltotriose-producing enzyme to act on the plant-base raw material.

The present technique further provides a method for reducing a saccharide in a plant-base food or beverage, the method comprising the step of allowing a cyclodextrin-producing enzyme to act on a plant-base raw material.

The method for reducing a saccharide in a plant-base food or beverage according to the present technique can also include a step of allowing a maltotriose-producing enzyme to act on the plant-base raw material.

In the present technique, "saccharide" is a general term for monosaccharides and disaccharides.

### Advantageous Effects of Invention

According to the present technique, it is possible to reduce saccharide in a plant-base food or beverage.

### Description of Embodiments

A preferred embodiment for carrying out the present invention will be described below. Note that the embodiment described below is an example of a typical embodiment of the present invention, and the scope of the present invention is not to be construed narrowly by this embodiment.

### 1. Enzymatic agent for reducing saccharide

The enzymatic agent for reducing a saccharide according to the present technique contains a cyclodextrin-producing enzyme as an active ingredient. Preferably, the enzymatic agent is substantially free of α-amylase. "Substantially free of α-amylase" means that the enzymatic agent is allowed to contain α-amylase to the extent that the objective can be achieved, that is, to the extent that the amount of a saccharide produced is not affected.

The content of α-amylase that does not affect the amount of a saccharide produced can be freely set depending on the type of a plant-base food or beverage to be manufactured, the type of raw materials used, etc. It can be set to preferably 0.01 U or less, more preferably 0.001 U or less, and even more preferably 0.0001 U or less per 1 g of a plant-base raw material used in the plant-base food or beverage to be manufactured.

### (1) Cyclodextrin-producing enzyme

The cyclodextrin-producing enzyme (EC 2.4.1.19) that can be used in the present technique is an enzyme that has an activity to produce cyclodextrin having a degree of polymerization of 6 to 8 using a polymer composed of α-1,4-linked glucose, such as starch, as a substrate. The cyclodextrin-producing enzyme that can be used in the present technique may be an enzyme that further has another action, so long as it has the activity of producing cyclodextrin.

In this technique, by allowing a cyclodextrin-producing enzyme to act on a plant-base raw material, the starch in the plant-base raw material can be liquefied and the saccharide (monosaccharides and disaccharides) in the manufactured plant-base food or beverage can be reduced. In addition, by allowing a cyclodextrin-producing enzyme to act on a plant-base raw material, off-flavors can be masked by the cyclodextrin in the manufactured plant-base food or beverage.

The origin of the cyclodextrin-producing enzyme that can be used in the present technique is not particularly limited, and examples thereof include cyclodextrin-producing enzymes derived from the genus Bacillus, such as Bacillus stearothermophilus, Bacillus megaterium, Bacillus circulans, Bacillus macerans, Bacillus ohbensis, and Bacillus clarkii; the genus Geobacillus, such as Geobacillus stearothermophilus; the genus Paenibacillus, such as Paenibacillus macerans; the genus Klebsiella, such as Klebsiella pneumoniae; the genus Thermoanaerobacter; and the genus Brevibacterium. One type of these cyclodextrin-producing enzymes may be used alone, or multiple types may be used in combination. Among these, from the viewpoint of improving the flavor of plant-base raw materials, the cyclodextrin-producing enzyme is preferably cyclodextrin glucanotransferase derived from Paenibacillus and/or cyclodextrin glucanotransferase derived from Geobacillus, and more preferably cyclodextrin glucanotransferase derived from Paenibacillus macerans and/or cyclodextrin glucanotransferase derived from Anoxybacillus caldiproteolyticus (including the one formerly known as Geobacillus stearothermophilus).

Here, "cyclodextrin-producing enzyme derived from Paenibacillus macerans" and/or "cyclodextrin-producing enzyme derived from Geobacillus stearothermophilus" means a cyclodextrin-producing enzyme produced by a microorganism (which may be a wild type or a mutant strain) classified as Paenibacillus macerans and/or Geobacillus stearothermophilus, or a cyclodextrin-producing enzyme obtained by a genetic engineering technique using a cyclodextrin-producing enzyme gene. Therefore, a recombinant produced by a host microorganism into which a cyclodextrin-producing enzyme gene (or a gene modified from said gene) obtained from Paenibacillus macerans and/or Geobacillus stearothermophilus has been introduced also falls under "cyclodextrin-producing enzyme derived from Paenibacillus macerans" and/or "cyclodextrin-producing enzyme derived from Geobacillus stearothermophilus".

The cyclodextrin-producing enzyme used in the present technique can be prepared from a culture solution of a microorganism from which the cyclodextrin-producing enzyme is derived. A specific preparation method includes a method of recovering the cyclodextrin-producing enzyme from the culture solution or microbial bodies of the microorganism. For example, when a cyclodextrin-producing enzyme-secreting microorganism is used, the microbial bodies can be recovered from the culture solution by filtration, centrifugation, or the like in advance as necessary, and the enzyme can then be separated and/or purified. When a cyclodextrin-producing enzyme non-secreting microorganism is used, the microbial bodies can be recovered from the culture solution in advance as necessary and then crushed by pressure treatment, ultrasonic treatment, or the like to expose an enzyme, and the enzyme can then be separated and/or purified. As the method for separating and/or purifying the enzyme, a known protein separation and/or purification method can be used without any particular limitation, and examples of the method include centrifugation, UF concentration, salting out, and various chromatography methods using ion exchange resins, etc. The separated and/or purified enzyme can be powdered by a drying method such as freeze-drying or drying under reduced pressure, or can be powdered by using an appropriate excipient and/or drying aid in the drying method. The separated and/or purified enzyme can also be liquefied by adding an appropriate additive and sterilizing by filtration.

In the present technique, commercially available cyclodextrin-producing enzymes can also be used, and preferred examples of commercially available products include Paenibacillus macerans-derived cyclodextrin glucanotransferase manufactured by Amano Enzyme Inc., and Geobacillus stearothermophilus-derived cyclodextrin glucanotransferase manufactured by Amano Enzyme Inc.

The content of the cyclodextrin-producing enzyme in the enzymatic agent for reducing a saccharide according to the present technique can be freely set as long as it does not impair the effect of the present technique. The content of the cyclodextrin-producing enzyme can be set to, for example, 0.01 U or more per 1 g of the plant-base raw material used in the plant-base food or beverage to be manufactured, and from the viewpoint of further enhancing the saccharide-reducing effect, it can be set to preferably 0.05 U or more, more preferably 0.1 U or more, even more preferably 0.3 U or more, and even more preferably 0.5 U or more.

The content of the cyclodextrin-producing enzyme can be set, for example, to 0.02 U or more per 1 g of polysaccharide (preferably starch) used in the plant-base food or beverage to be manufactured, and from the viewpoint of further enhancing the saccharide reduction effect, can be set preferably to 0.08 U or more, more preferably to 0.2 U or more, even more preferably to 0.5 U or more, and even more preferably to 0.8 U or more.

The upper limit of the amount of cyclodextrin-producing enzyme contained is not particularly limited as long as it does not impair the effects of the present technique, but can be set to, for example, 300 U or less, 200 U or less, 100 U or less, 50 U or less, 40 U or less, or 30 U or less per 1 g of a plant-base raw material used in the plant-base food or beverage to be manufactured.

In addition, the upper limit of the cyclodextrin-producing enzyme content can be set to, for example, 500 U or less, 300 U or less, 150 U or less, 80 U or less, 60 U or less, or 40 U or less per 1 g of polysaccharide (preferably starch) used in the plant-base food or beverage to be manufactured.

In the present technique, the activity of a cyclodextrin-producing enzyme is a value measured by the measurement method described in the Examples below.

### (2) Others

As long as the enzymatic agent for reducing a saccharide according to the present technique contains the above-mentioned cyclodextrin-producing enzyme, it may be composed of only the above-mentioned cyclodextrin-producing enzyme, or may contain one or more types of other components selected freely as long as the effect of the present technique is not impaired. Examples of other components that can be used include excipients, pH adjusters, colorants, flavoring agents, disintegrants, lubricants, stabilizers, and other components that are normally used in formulations. Furthermore, components having known or future functions can be used in combination as appropriate depending on the purpose.

### (3) Maltotriose-producing enzyme synthase

The enzymatic agent for reducing a saccharide according to the present technique can also contain a maltotriose-producing enzyme. The maltotriose-producing enzyme that can be used in the present technique is an enzyme that acts on starch and has an activity of mainly producing maltotriose. The maltotriose-producing enzyme that can be used in the present technique may also be an enzyme that has other actions as long as it has the activity of producing maltotriose.

In this technique, by allowing a maltotriose-producing enzyme to act on plant-base raw materials that have been treated with a cyclodextrin-producing enzyme, it is possible to increase the amount of trisaccharides while maintaining the amount of saccharides (monosaccharides and disaccharides) in the manufactured plant-base food or beverage at a low level, thereby imparting a moderate sweetness.

The origin of the maltotriose-producing enzyme that can be used in the present technique is not particularly limited, and examples thereof include maltotriose-producing enzymes derived from organisms of the genus Streptomyces, Bacillus, Microbacterium, and Cellulosimicrobium. One type of these maltotriose-producing enzymes may be used alone, or multiple types may be used in combination. Among these maltotriose-producing enzymes, preferred are maltotriose-producing enzymes derived from organisms of the genus Microbacterium or Cellulosimicrobium, and more preferred are maltotriose-producing enzymes derived from Microbacterium sp. Specifically, enzymes that show substrate specificity acting on amylose, amylopectin, glycogen, and starch can be used.

Here, "maltotriose-producing enzyme derived from Microbacterium sp." means a maltotriose-producing enzyme produced by a microorganism (whether a wild type or a mutant type) classified as Microbacterium sp., or a maltotriose-producing enzyme obtained by a genetic engineering technique using a maltotriose-producing enzyme gene. Therefore, a recombinant produced by a host microorganism into which a maltotriose-producing enzyme gene obtained from Microbacterium sp. (or a gene obtained by modifying said gene) has been introduced also falls under "maltotriose-producing enzyme derived from Microbacterium sp.".

The maltotriose-producing enzyme used in the present technique can be prepared from the culture solution of a microorganism from which the maltotriose-producing enzyme is derived. A specific preparation method includes a method of recovering the maltotriose-producing enzyme from the culture solution or microbial bodies of the microorganism. For example, when a maltotriose-producing enzyme-secreting microorganism is used, the microbial bodies can be recovered from the culture solution by filtration, centrifugation, or the like in advance as necessary and then the enzyme can be separated and/or purified. In addition, when a maltotriose-producing enzyme non-secreting microorganism is used, the microbial bodies can be recovered from the culture solution in advance as necessary and then crushed by pressure treatment, ultrasonic treatment, or the like to expose an enzyme, and then the enzyme can be separated and/or purified. As the method for separating and/or purifying the enzyme, a known protein separation and/or purification method can be used without any particular limitation, and examples thereof include centrifugation, UF concentration, salting out, and various chromatography methods using ion exchange resins, etc. The separated and/or purified enzyme can be powdered by a drying method such as freeze-drying or drying under reduced pressure, or can be powdered by using an appropriate excipient and/or drying aid in the drying method. The separated and/or purified enzyme can also be liquefied by adding an appropriate additive and sterilizing by filtration.

In the present technique, a commercially available product can be used as the maltotriose-producing enzyme, and a preferred example of a commercially available product is a maltotriose-producing enzyme manufactured by Amano Enzyme Inc.

### 2. Plant-base food or beverage

The plant-base food or beverage according to the present technique is a plant-base food or beverage manufactured using the above-mentioned enzymatic agent for reducing a saccharide. Specific examples of the plant-base beverage include oat beverage, more specifically oat milk (also called "oats milk"). Specific examples of the plant-base food include plant-base yogurt.

The amount of saccharides (monosaccharides and disaccharides) contained in the plant-base food or beverage according to the present technique is not particularly limited. For example, the amount of a saccharide contained in the plant-base food or beverage is 5 mg or less, 4 mg or less, preferably 3 mg or less, more preferably 2 mg or less, and even more preferably 1.5 mg or less per 1 g of the plant-base food or beverage.

The plant-base food or beverage according to the present technique may have a reduced amount of saccharides (monosaccharides and disaccharides) and an increased amount of cyclodextrin. The increased amount of cyclodextrin can mask off-flavors. The amount of cyclodextrin contained in the plant-base food or beverage according to the present technique is also not particularly limited. The amount of cyclodextrin contained in the plant-base food or beverage is, for example, 1 mg or more, preferably 2 mg or more, more preferably 3 mg or more, even more preferably 4 mg or more, and even more preferably 5 mg or more, as the total amount of α-cyclodextrin, per 1 g of the plant-base food or beverage.

In addition, the ratio of cyclodextrin to saccharides (monosaccharides + disaccharides) (cyclodextrin/saccharides (monosaccharides + disaccharides)) is not particularly limited, but in order to obtain both the saccharide reducing effect and the masking effect, it is, for example, 3 or more, preferably 4 or more, and more preferably 5 or more.

When the plant-base food or beverage according to the present technique is subjected to a maltotriose-producing enzyme treatment step, the amount of saccharides (monosaccharides and disaccharides) is reduced and the amount of trisaccharides is increased. The increase in trisaccharides can impart sweetness. The amount of trisaccharides contained in the plant-base food or beverage according to the present technique is also not particularly limited. The amount of trisaccharides contained in the plant-base food or beverage is, for example, 2 mg or more, preferably 4 mg or more, more preferably 6 mg or more, even more preferably 8 mg or more, and even more preferably 10 mg or more per 1 g of the plant-base food or beverage.

### 3. Method for manufacturing plant-base food or beverage, and method for reducing saccharide in plant-base food or beverage

The method for manufacturing a plant-base food or beverage according to the present technique is a method including a step of allowing a cyclodextrin-producing enzyme to act in a liquefaction process of a plant-base raw material (hereinafter also referred to as a "cyclodextrin-producing enzyme action step"). The method for reducing a saccharide in a plant-base food or beverage according to the present technique is a method including a step of allowing a cyclodextrin-producing enzyme to act on a plant-base raw material (hereinafter also referred to as a "cyclodextrin-producing enzyme action step"). In addition, depending on the type of a plant-base food or beverage, it is also possible to carry out general food manufacturing steps before, after, or simultaneously with each step, as long as the effects of the present technique are not impaired. In the above-mentioned method for manufacturing a plant-base food or beverage and the method for reducing a saccharide in a plant-base food or beverage, further a maltotriose-producing enzyme action step and a recovery step can also be carried out.

In the manufacturing method and saccharide reducing method according to the present technique, it is preferable not to carry out a step of allowing α-amylase to act on a plant-base raw material for the purpose of liquefying starch (hereinafter also referred to as "α-amylase action step"). It is possible to carry out the α-amylase action step as long as it does not affect the amount of a saccharide produced, but, when the α-amylase action step is carried out, it is preferable to carry out the α-amylase action step before the cyclodextrin-producing enzyme action step described below, or simultaneously with the cyclodextrin-producing enzyme action step. The amount of α-amylase added when carrying out the α-amylase action step can be freely set as long as it does not impair the effect of the present technique. The specific amount of α-amylase added in the present technique is the same as the content when α-amylase is contained in the above-mentioned enzymatic agent for reducing a saccharide to the extent that it does not affect the amount of a saccharide produced, so a description thereof will be omitted here. Each step will be described in detail below.

### (1) Plant-base raw material

The plant-base raw materials that can be used in the present technique are not particularly limited in origin, type, etc. of the plant-base raw material, as long as they do not impair the effects of the present technique, and can be freely selected according to the intended plant-base food or beverage. Examples thereof include beans such as soy beans, green peas, lentils, chickpeas, black beans, broad beans, mung beans, lupine beans, kidney beans, etc.; cereals such as wheat, barley, oats, rice, rye, buckwheat, barnyard millet, millet, teff, etc.; nuts such as almonds, coconuts, peanuts, cashew nuts, hazelnuts, pecan nuts, macadamia nuts, pistachios, walnuts, Brazil nuts, pili nuts, chestnuts, sesame seeds, pine nuts, etc.; hemp seeds (industrial hemp), chia seeds, quinoa, amaranth, canary seeds, flaxseed, etc. In the present technique, one type of these may be used alone, or two types or more may be used in combination. Among these raw materials, preferred are cereals, and more preferred are oats.

In the present technique, the state of the plant-base raw material when subjected to various enzyme treatments is not particularly limited as long as it does not impair the effects of the present technique, but preferably, the plant-base raw material is in a liquid, slurry, or paste form.

The plant-base raw material that can be used in the present technique contains carbohydrates. The content of the plant-base carbohydrates contained in the plant-base raw material (based on the weight of the plant-base raw material in a dry state) is not particularly limited, but may be, for example, 20% by weight or more. Preferably, it is 30% by weight or more, more preferably 40% by weight or more, and even more preferably 50% by weight or more. The upper limit of the content range is not particularly limited, but may be, for example, 90% by weight or less, 80% by weight or less, or 70% by weight or less.

In the present technique, it is preferable to use a plant-base raw material that has not been treated with α-amylase when carrying out the cyclodextrin-producing enzyme reaction step described below. When starch contained in the plant-base raw material is liquefied using a cyclodextrin-producing enzyme without α-amylase treatment, it is possible to reduce the amount of a saccharide in the plant-base food or beverage after liquefaction.

In the present technique, the plant-base raw material used in the maltotriose-producing enzyme reaction step described below is not particularly limited, but it is preferable to use a plant-base raw material treated with a cyclodextrin-producing enzyme. It is more preferable to use a plant-base raw material in which the cyclodextrin-producing enzyme has been inactivated after treatment with the cyclodextrin-producing enzyme. The maltotriose-producing enzyme treatment increases trisaccharides in a plant-base food or beverage with a reduced saccharide, thereby imparting sweetness.

### (2) Cyclodextrin-producing enzyme action step

The cyclodextrin-producing enzyme action step is a step in which a cyclodextrin-producing enzyme is allowed to act on a plant-base raw material. The amount of cyclodextrin-producing enzyme added in the cyclodextrin-producing enzyme action step can be freely set as long as it does not impair the effect of the present technique. The specific amount of cyclodextrin-producing enzyme added in the present technique is the same as the content of cyclodextrin-producing enzyme in the above-mentioned enzymatic agent for reducing a saccharide, so a description thereof will be omitted here.

Various conditions for the cyclodextrin-producing enzyme action step can be freely set as long as they do not impair the effects of the present technique. For example, the pH, temperature, action time, and the like can be set according to the physicochemical properties of the cyclodextrin-producing enzyme used, such as the optimum pH, stable pH range, optimum temperature, and temperature stability. The pH can be set, for example, to pH 5.0 to 8.0, preferably pH 5.5 to 7.5, and more preferably pH 5.5 to 7.0. The temperature can be set, for example, to 30°C to 80°C, preferably 40°C to 75°C, and more preferably 50°C to 70°C. The action time can be set, for example, to 10 minutes to 48 hours, preferably 30 minutes to 24 hours, and more preferably 1 hour to 12 hours. The optimal reaction conditions can be determined through preliminary experiments.

### (3) Maltotriose-producing enzyme action step

The maltotriose-producing enzyme action step is a step of allowing a maltotriose-producing enzyme to act on a plant-base raw material. The amount of the maltotriose-producing enzyme added in the maltotriose-producing enzyme action step can be freely set as long as it does not impair the effects of the present technique. In the present technique, the specific amount of the maltotriose-producing enzyme added can be set to preferably 0.5 U or more, more preferably 1 U or more, and even more preferably 5 U or more as the amount of the maltotriose-producing enzyme used per 1 g of the plant-base raw material, from the viewpoint of further enhancing the saccharide reducing effect and the sweetness enhancement effect. The upper limit of the range of the amount of the maltotriose-producing enzyme used per 1 g of the plant-base raw material is not particularly limited, but can be set to, for example, 1500 U or less, 300 U or less, 150 U or less, 100 U or less, 50 U or less, 20 U or less, or 15 U or less.

Various conditions for the maltotriose-producing enzyme action step can be freely set as long as they do not impair the effects of the present technique. For example, the pH, temperature, action time, etc. can be set according to the physicochemical properties of the maltotriose-producing enzyme used, such as the optimum pH, stable pH range, optimum temperature, and temperature stability. The pH can be set, for example, to pH 5.0 to 8.0, preferably pH 5.5 to 7.5, and more preferably pH 6.0 to 7.0. The temperature can be set, for example, to 30°C to 80°C, preferably 40°C to 75°C, and more preferably 45°C to 65°C. The action time can be set, for example, to 10 minutes to 12 hours, preferably 30 minutes to 6 hours, and more preferably 1 hour to 3 hours. The optimal reaction conditions can be determined through preliminary experiments.

### (4) Recovery step

The recovery step is a step of recovering the plant-base food or beverage that has been subjected to the cyclodextrin-producing enzyme action step. When the above-mentioned maltotriose-producing enzyme action step is performed, the recovery step recovers the plant-base food or beverage that has been subjected to the cyclodextrin-producing enzyme action step and the maltotriose-producing enzyme action step. As for the specific recovery method, one type of general recovery methods used in the manufacturing of a plant-base food or beverage can be used alone, or two types or more can be freely combined and used, depending on the type of the plant-base food or beverage to be manufactured.

The plant-base food or beverage that has been subjected to the cyclodextrin-producing enzyme action step is characterized by having a reduced amount of saccharides (monosaccharides + disaccharides). Specifically, for example, the content of saccharides (monosaccharides + disaccharides) in the plant-base food or beverage that has been subjected to the cyclodextrin-producing enzyme action step is 5 mg or less per 1 g of the plant-base food or beverage. In other words, the present technique can perform a recovery step of recovering a plant-base food or beverage that has saccharides (monosaccharides + disaccharides) content of 5 mg or less per 1 g of the plant-base food or beverage.

In addition, in the present technique, by carrying out the maltotriose-producing enzyme action step, it is possible to increase the trisaccharides in a plant-base food or beverage with reduced saccharides (monosaccharides + disaccharides), thereby imparting sweetness. Specifically, for example, the content of trisaccharides in the plant-base food or beverage that has been subjected to the maltotriose-producing enzyme action step is 2 mg or more per 1 g of the plant-base food or beverage. In other words, in the present technique, a recovery step can be carried out to recover a plant-base food or beverage with a trisaccharide content of 2 mg or more per 1 g of the plant-base food or beverage.

To summarize the above, the present technique can perform a recovery step to recover a plant-base food or beverage having saccharides (monosaccharides + disaccharides) content of 5 mg or less per 1 g of the plant-base food or beverage and a trisaccharide content of 2 mg or more per 1 g of the plant-base food or beverage.

One aspect of the method for manufacturing a plant-base food or beverage and the method for reducing a saccharide of the present technique includes the following steps (1) and (2). Note that an enzyme inactivation step may be added after step (2).
(1) A step of preparing a plant-base raw material containing a carbohydrate.
(2) A step of treating the prepared raw material with a cyclodextrin-producing enzyme.

One aspect of the method for manufacturing a plant-base food or beverage and the method for reducing a saccharide of the present technique includes the following steps (1) to (3). Note that an enzyme inactivation step may be added after step (2).
(1) A step of preparing a plant-base raw material containing a carbohydrate.
(2) A step of treating the prepared raw material with a cyclodextrin-producing enzyme without treating it with α-amylase.
(3) A step of recovering a plant-base food or beverage having saccharides (monosaccharides + disaccharides) content of 5 mg or less per 1 g of the plant-base food or beverage.

In the step (3), it is also possible to recover a plant-base food or beverage having a trisaccharide content of 2 mg or more per 1 g of the plant-base food or beverage.

One aspect of the method for manufacturing a plant-base food or beverage and the method for reducing a saccharide of the present technique includes the following steps (1) to (3).
(1) A step of preparing a plant-base raw material containing a carbohydrate.
(2) A step of treating the prepared raw material with a cyclodextrin-producing enzyme.
(3) A step of treating the prepared raw material with a maltotriose-producing enzyme.

Note that step (3) may be carried out simultaneously with step (2) or after step (2). Preferably, step (3) is carried out after step (2), and more preferably, an enzyme inactivation step may be added after step (2). That is, it is preferable to include the following steps (1) to (4).
(1) A step of preparing a plant-base raw material containing a carbohydrate.
(2) A step of treating the prepared raw material with a cyclodextrin-producing enzyme.
(3) A step of inactivating the cyclodextrin-producing enzyme in the raw material treated in the step (2).
(4) A step of treating the raw material obtained in step (3) with a maltotriose-producing enzyme.

Another aspect of the method for manufacturing a plant-base food or beverage and the method for reducing a saccharide of the present technique includes the following steps (1) and (2). Note that an enzyme inactivation step may be added after step (1) and/or step (2).
(1) A step of preparing a carbohydrate-containing plant-base raw material treated with a cyclodextrin-producing enzyme.
(2) A step of treating the prepared raw material with maltotriose-producing enzyme.

### EXAMPLES

The present invention will be described in more detail below with reference to examples. Note that the examples described below are representative examples of the present invention, and the scope of the present invention should not be construed as being narrowed by these examples.

### 1. Enzyme used

Cyclodextrin-producing enzyme
CGT-1: Cyclodextrin glucanotransferase derived from Paenibacillus macerans, manufactured by Amano Enzyme Inc.
CGT-2: Cyclodextrin glucanotransferase derived from Geobacillus stearothermophilus, manufactured by Amano Enzyme Inc.

Maltotriose-producing enzyme
Maltotriose-producing enzyme derived from Microbacterium sp., manufactured by Amano Enzyme Inc.

### 2. Enzyme activity measurement method

### [Method for measuring cyclodextrin-producing enzyme activity]

One gram of potato starch was dissolved in 100 mL of distilled water (adjusted to pH 5.5 with dilute acetic acid) and completely gelatinized. Ten milliliters of the gelatinized solution was measured, and 1 mL of an enzyme solution was added and mixed. The mixture was precisely incubated at 40°C for 10 minutes, and the reaction was stopped with 0.1 N hydrochloric acid. Ten milliliters of iodine/potassium iodide test solution (0.4 mmol/L) was added to 1 mL of this reaction solution and mixed to obtain a test solution. The iodine/potassium iodide test solution was prepared by dissolving 10.0 g of potassium iodide and 1.0 g of iodine in water to make 100 mL, and then diluting 200 times with water. The absorption wavelength of the mixed solution at 660 nm was then measured. As a control, potato starch that was not treated with an enzyme was also prepared as a mixed solution in the same manner, and the absorption wavelength at 660 nm was measured. The amount of the enzyme that reduces the blue iodine color of starch by 1% in 1 minute was defined as 1 unit (1 U).

### [Method for measuring maltotriose-producing enzyme activity]

The enzyme was allowed to act on the soluble starch as a substrate, and the resulting reducing saccharide was colorimetrically quantified by the Somogyi-Nelson method. Zero point five milliliters of the soluble starch solution and 0.4 mL of 0.1 mol/L acetic acid-sodium acetate buffer (pH 6.0) (containing 0.05 mol/L CaCl₂) were weighed into a 50 mL Nessler tube, shaken well, and left at 40±0.5°C for 10 to 15 minutes, after which 0.1 mL of the sample solution was added and immediately shaken. This solution was left at 40±0.5°C for exactly 15 minutes, after which 1 mL of an alkaline copper test solution was added, shaken, plugged, heated in a boiling water bath for exactly 20 minutes, and immediately cooled. After cooling, 1 mL of Nelson's solution was added, shaken well until the red precipitate of cuprous oxide was completely dissolved, then left at room temperature for 20 minutes, and 22 mL of water was added and shaken well. The absorbance of this solution at a wavelength of 520 nm was measured using water as a control. The production of a reducing saccharide equivalent to 1 µmol of glucose per minute was defined as one unit.

### 3. Experimental example

### <Experimental Example 1>

In Experimental Example 1, the effect of reducing a saccharide by a cyclodextrin-producing enzyme was examined.

### (1) Experimental method

Twelve point five grams of oat flour (PREMIUM OAT FLOUR, the carbohydrate content 67.7 g/100 g, Slow Food Kitchen) was added to 100 mL of water to suspend it, and cyclodextrin-producing enzyme was added to the mixture so that the amount of the enzyme was 0 to 30 U relative to the raw material. After the enzyme was added, the enzyme was allowed to act for 2 to 24 hours while stirring in a 60°C water bath to liquefy the starch contained in the oat flour, and the enzyme was then inactivated by treating at 95°C for 10 minutes. One hundred ninety microliters of 1 mol/L trichloroacetic acid was added to 1.5 mL of the enzyme-inactivated oat solution, and the mixture was allowed to stand at room temperature for 10 minutes. After 10 minutes, the mixture was centrifuged (12,000 rpm, 10 minutes) to recover the supernatant. The recovered supernatant was suspended in an equal amount of diethyl ether added. The mixture was centrifuged (12,000 rpm, 10 minutes), the organic solvent layer was discarded, and the aqueous layer was recovered. This operation was repeated twice. The recovered aqueous layer was added with an equal amount of 99.5% ethanol (for high performance liquid chromatography, manufactured by FUJIFILM Wako Pure Chemical Corp.), stirred, filtered through a membrane filter, and subjected to analysis. For the quantification of α-cyclodextrin, a sample was also prepared by carrying out the same treatment, but adding a step of adding 1% glucoamylase and allowing it to act at 60°C for 2 hours after the cyclodextrin-producing enzyme treatment. The analysis was carried out using high performance liquid chromatography, and the columns used were MCL GEL CK04S manufactured by Mitsubishi Chemical Corporation and TSK gel Amide-80 manufactured by Tosoh Corporation. The amount of each component produced was quantified from the peak area. As a comparative example, the same operation was carried out using α-amylase instead of the cyclodextrin-producing enzyme.

### (2) Result

The results are shown in Tables 1 and 2 below.

**[Table 1]**

| | Unit | Comparative Example | Example | | | | | | | | | | | | | | |
|---|---|---|---|---|---|---|---|---|---|---|---|---|---|---|---|---|---|
| | | 1 | 1 | 2 | 3 | 4 | 5 | 6 | 7 | 8 | 9 | 10 | 11 | | 13 | 14 | 15 |
| Amount of α-amylase added | %-raw material | 0.05 | 0 | | | 0 | | | 0 | | | 0 | | | 0 | | |
| Amount of CGT-1 added | U/g-raw material | 0 | 0.6 | | | 3 | | | 6 | | | 18 | | | 30 | | |
| Reaction time | hour | 2 | 2 | 4 | 24 | 2 | 4 | 24 | 2 | 4 | 24 | 2 | 4 | 24 | 2 | 4 | 24 |
| α-CD | mg/mL | 0 | 2.2 | 3.2 | 2.2 | 4.6 | 5.3 | 3.8 | 5.4 | 6.4 | 4.2 | 7 | 7.3 | 5.5 | 6 | 6.6 | 5 |
| Maltose | mg/mL | 4 | 0 | 0 | 0.2 | 0 | 0 | 0.6 | 0 | 0.7 | 0.8 | 0.9 | 1.1 | 1.2 | 0.9 | 1.1 | 1.2 |
| Glucose | mg/mL | 1.4 | 0 | 0 | 0 | 0 | 0 | 0 | 0 | 0 | 0 | 0 | 0 | 0 | 0 | 0 | 0 |
| Total saccharide (monosaccharide + disaccharide) | mg/mL | 5.4 | 0 | 0 | 0.2 | 0 | 0 | 0.6 | 0 | 0.7 | 0.8 | 0.9 | 1.1 | 1.2 | 0.9 | 1.1 | 1.2 |

| | | | | | | | | | | | | | | | | | |
|---|---|---|---|---|---|---|---|---|---|---|---|---|---|---|---|---|---|
| α-CD: α-cyclodextrin | | | | | | | | | | | | | | | | | |

**[Table 2]**

| | Unit | Comparative Example | Example | | | | | | | | | | | | | | |
|---|---|---|---|---|---|---|---|---|---|---|---|---|---|---|---|---|---|
| | | 1 | 16 | 17 | 18 | 19 | 20 | 21 | 22 | 23 | 24 | 25 | 26 | 27 | 28 | 29 | 30 |
| Amount of α-amylase added | %-raw material | 0.05 | 0 | | | 0 | | | 0 | | | 0 | | | 0 | | |
| Amount of CGT-2 added | U/g-raw material | 0 | 0.4 | | | 2 | | | 4 | | | 12 | | | 20 | | |
| Reaction time | hour | 2 | 2 | 4 | 24 | 2 | 4 | 24 | 2 | 4 | 24 | 2 | 4 | 24 | 2 | 4 | 24 |
| α-CD | mg/mL | 0 | 2.7 | 4.5 | 6 | 5.9 | 8.1 | 6.4 | 6.8 | 8.4 | 6.4 | 6.8 | 7.4 | 5.5 | 6.4 | 7 | 4.1 |
| Maltose | mg/mL | 4 | 0 | 0 | 0 | 0 | 0.4 | 0 | 0 | 0 | 0 | 0 | 0.1 | 0 | 0.4 | 0.2 | 0 |
| Glucose | mg/mL | 1.4 | 0.1 | 0.1 | 0 | 0 | 0 | 0 | 0 | 0 | 0 | 0 | 0.3 | 0 | 0 | 0.2 | 0.2 |
| Total saccharide (monosaccharide + disaccharide) | mg/mL | 5.4 | 0.1 | 0.1 | 0 | 0 | 0.4 | 0.0 | 0 | 0 | 0 | 0 | 0.4 | 0 | 0.4 | 0.3 | 0.2 |

| | | | | | | | | | | | | | | | | | |
|---|---|---|---|---|---|---|---|---|---|---|---|---|---|---|---|---|---|
| α-CD: α-cyclodextrin | | | | | | | | | | | | | | | | | |

### (3) Observation

As shown in Tables 1 and 2, the saccharide content of the oat milk treated with α-amylase in Comparative Example 1 was 5.4 mg/mL. On the other hand, as shown in Examples 1 to 30, it was confirmed that the amount of the saccharide could be reduced to 1.2 mg/mL or less by using a cyclodextrin-producing enzyme.

### <Experimental Example 2>

In Experimental Example 2, the saccharide reducing effect and viscosity reducing effect of the cyclodextrin-producing enzyme were examined.

### (1) Experimental method

Twelve point five grams of oat flour (PREMIUM OAT FLOUR, the carbohydrate content 67.7 g/100 g, Slow Food Kitchen) was added to 100 mL of water to suspend it, and cyclodextrin-producing enzyme was added to the mixture so that the amount was 0 to 18 U relative to the raw material. After the enzyme was added, the enzyme was allowed to act for 2 or 4 hours while stirring in a 60°C water bath to liquefy the starch contained in the oat flour. The enzyme was inactivated by treating at 95°C for 10 minutes. One hundred ninety microliters of 1 mol/L trichloroacetic acid was added to 1.5 mL of the enzyme-inactivated oat solution, and the mixture was left to stand at room temperature for 10 minutes. After 10 minutes, the mixture was centrifuged (12,000 rpm, 10 minutes) and the supernatant was recovered. The recovered supernatant was suspended in an equal amount of diethyl ether added. The mixture was centrifuged (12,000 rpm, 10 minutes), the organic solvent layer was discarded, and the aqueous layer was recovered. This operation was repeated twice. The recovered aqueous layer was added with an equal amount of 99.5% ethanol (for high performance liquid chromatography, manufactured by FUJIFILM Wako Pure Chemical Corp.), stirred, and filtered through a membrane filter, which was then subjected to analysis. High performance liquid chromatography was used for the analysis, and columns used were MCL GEL CK04S manufactured by Mitsubishi Chemical Corporation and TSK gel Amide -80 manufactured by Tosoh Corporation. The amounts of glucose and maltose produced were quantified from the peak area. In addition, the viscosity of the enzyme-inactivated oat solution was measured. Fifty milliliters of the enzyme-inactivated oat solution was placed in a tube, and the viscosity was measured by heating to 60°C using a viscometer (TVB-15 type viscometer, rotor No. 4 or No. 2, Toki Sangyo Co., Ltd.). As a comparative example, the same operation was performed using α-amylase instead of the cyclodextrin-producing enzyme. As a reference example, the same operation was performed without adding any enzyme.

### (2) Result

The results are shown in Table 3 below.

**[Table 3]**

| | Unit | Reference Example | | Comparative Example | | Example | | | | | | | | | | | |
|---|---|---|---|---|---|---|---|---|---|---|---|---|---|---|---|---|---|
| | | 1 | 2 | 2 | 3 | 31 | 32 | 33 | 34 | 35 | 36 | 37 | 38 | 39 | 40 | 41 | 42 |
| Amount of α-amylase added | %-raw material | 0 | | 0.05 | | 0 | | 0 | | 0 | | 0 | | 0 | | 0 | |
| Amount of CGT-1 added | U/g-raw material | 0 | | 0 | | 0.6 | | 6 | | 18 | | 0 | | 0 | | 0 | |
| Amount of CGT-2 added | U/g-raw material | 0 | | 0 | | 0 | | 0 | | 0 | | 0.4 | | 4 | | 12 | |
| Reaction time | hour | 2 | 4 | 2 | 4 | 2 | 4 | 2 | 4 | 2 | 4 | 2 | 4 | 2 | 4 | 2 | 4 |
| Viscosity | Pa · s | 46.5 | 37.7 | 0.01 | 0.01 | 8.7 | 2.4 | 0.1 | 0.05 | 0.04 | 0.03 | 0.9 | 0.7 | 0.1 | 0.1 | 0.08 | 0.08 |
| Maltose | mg/mL | - | - | 4.9 | 5.3 | 0 | 0 | 0 | 0 | 0 | 0 | 0 | 0 | 0.3 | 0.4 | 0.4 | 0.6 |
| Glucose | mg/mL | - | - | 1.5 | 1.5 | 0.1 | 0.1 | 0 | 0.1 | 0.1 | 0.1 | 0 | 0 | 0 | 0.0 | 0 | 0.2 |
| Total saccharide (monosaccharide + disaccharide) | mg/mL | - | - | 6.3 | 6.8 | 0.1 | 0.1 | 0 | 0.1 | 0.1 | 0 | 0 | 0 | 0 | 0.4 | 0.4 | 0.8 |

### (3) Observation

As shown in Table 3, the α-amylase-treated oat milk of Comparative Examples 2 and 3 had a significantly lowered viscosity, while the saccharide content was 6 to 7 mg/mL. On the other hand, as shown in Examples 31 to 42, it was confirmed that the use of a cyclodextrin-producing enzyme could reduce the amount of a saccharide to 1 mg/mL or less while still lowering the viscosity to a certain degree.

### <Experimental Example 3>

The enzyme-treated oat milk obtained above has a significantly reduced amount of saccharides (monosaccharides + disaccharides), and as a result, its sweetness is also reduced. Therefore, in order to reduce the amount of saccharides (monosaccharides + disaccharides) while imparting sweetness, the effect of reducing a saccharide and producing trisaccharides by using cyclodextrin-producing enzyme and maltotriose-producing enzyme in combination was investigated.

### (1) Experimental method

Twelve point five grams of oat flour (PREMIUM OAT FLOUR, the carbohydrate content 67.7 g/100 g, Slow Food Kitchen) was suspended in 100 mL of water, and cyclodextrin-producing enzyme (CGT-1: 6 U/g-raw material, CGT-2: 4 U/g-raw material) and maltotriose-producing enzyme (12 U/g-raw material, 36 U/g-raw material, 60 U/g-raw material) were added to the raw material. After the enzyme addition, the enzyme was allowed to act for 2 hours while stirring in a 60°C water bath, and then the enzyme was inactivated by treating at 95°C for 10 minutes. One hundred ninety microliters of 1 mol/L trichloroacetic acid was added to 1.5 mL of the enzyme-inactivated oat solution, and the mixture was left to stand at room temperature for 10 minutes. After 10 minutes, the mixture was centrifuged (12,000 rpm, 10 minutes) and the supernatant was recovered. The recovered supernatant was suspended in an equal amount of diethyl ether added. Centrifugation (12,000 rpm, 10 minutes) was performed, the organic solvent layer was discarded, and the aqueous layer was recovered. This procedure was repeated twice. The recovered aqueous layer was added with an equal amount of 99.5% ethanol (for high performance liquid chromatography, manufactured by FUJIFILM Wako Pure Chemical Corp.), stirred, and filtered through a membrane filter, and the resultant was subjected to analysis. Analysis was performed using high performance liquid chromatography, and columns used were MCL GEL CK04S manufactured by Mitsubishi Chemical Corporation and TSK gel Amide-80 manufactured by Tosoh Corporation. The amount of each component produced was quantified from the peak area.

### (2) Result

The results are shown in Table 4 below.

**[Table 4]**

| | Unit | Example | | | | |
|---|---|---|---|---|---|---|
| | | 43 | 44 | 45 | 46 | 47 |
| Amount of CGT-1 added | U/g-raw material | 6 | 6 | 6 | 0 | 0 |
| Amount of CGT-2 added | U/g-raw material | 0 | 0 | 0 | 4 | 4 |
| Amount of maltotriose-producing enzyme added | U/g-raw material | 12 | 36 | 60 | 12 | 36 |
| Reaction time | hour | 2 | 2 | 2 | 2 | 2 |
| Total saccharide (monosaccharide + disaccharide) | mg/mL | 0.6 | 1.9 | 3.3 | 0.3 | 2.8 |
| Trisaccharide | mg/mL | 0.8 | 3.8 | 7.7 | 0.8 | 2.8 |

### (3) Observation

As shown in Table 4, the production of trisaccharides could be confirmed in the oat milk obtained by using a cyclodextrin-producing enzyme and a maltotriose-producing enzyme in combination. Therefore, it was found that the combined use of cyclodextrin-producing enzyme and maltotriose-producing enzyme can reduce saccharides (monosaccharides + disaccharides) and increase trisaccharides, compared to Comparative Example 1 of Experimental Example 1.

### <Experimental Example 4>

In Experiment 4, the effect of reducing a saccharide and the effect of producing trisaccharides when treatment with a maltotriose-producing enzyme was carried out after treatment with a cyclodextrin-producing enzyme were examined.

### (1) Experimental method

Twelve point five grams of oat flour (PREMIUM OAT FLOUR, the carbohydrate content 67.7 g/100 g, Slow Food Kitchen) was added to 100 mL of water to suspend it, and cyclodextrin-producing enzyme (CGT-1: 6 U/g-raw material, CGT-2: 4 U/g-raw material) was added to the raw material. After the enzyme was added, the enzyme was allowed to act for 2 hours while stirring in a 60°C water bath. After cooling to 50°C, maltotriose-producing enzyme (1.2 U/g-raw material, 6 U/g-raw material, 12 U/g-raw material, 36 U/g-raw material) was added. After the enzyme was added, the enzyme was allowed to act for 2 hours while stirring in a 50°C water bath, and then the enzyme was inactivated by treating at 95°C for 10 minutes. One hundred ninety microliters of 1 mol/L trichloroacetic acid was added to 1.5 mL of the enzyme-inactivated oat solution, and the mixture was allowed to stand at room temperature for 10 minutes. After 10 minutes, the mixture was centrifuged (12,000 rpm, 10 minutes) to recover the supernatant. The recovered supernatant was suspended in an equal amount of diethyl ether added. The mixture was centrifuged (12,000 rpm, 10 minutes), the organic solvent layer was discarded, and the aqueous layer was recovered. This procedure was repeated twice. The recovered aqueous layer was added with an equal amount of 99.5% ethanol (for high performance liquid chromatography, manufactured by FUJIFILM Wako Pure Chemical Corp.), stirred, and filtered through a membrane filter for analysis. The analysis was performed using high performance liquid chromatography, and columns used were MCL GEL CK04S manufactured by Mitsubishi Chemical Corporation and TSK gel Amide-80 manufactured by Tosoh Corporation. The amount of each component produced was quantified from the peak area.

### (2) Result

The results are shown in Table 5 below.

**[Table 5]**

| | Unit | Example | |
|---|---|---|---|
| | | 48 | 49 |
| Amount of CGT-1 added | U/g-raw material | 6 | 6 |
| Amount of CGT-2 added | U/g-raw material | 0 | 0 |
| Amount of maltotriose-producing enzyme added | U/g-raw material | 1.2 | 6 |
| Total saccharide (monosaccharide + disaccharide) | mg/mL | 1.6 | 4.6 |
| Trisaccharide | mg/mL | 3.1 | 10.1 |

### (3) Observation

As shown in Table 5, it was found that when the maltotriose-producing enzyme was allowed to act after the cyclodextrin-producing enzyme treatment, the amount of trisaccharide produced was greater than when both enzymes were allowed to act simultaneously.

### <Experimental Example 5>

In Experiment 5, the effect of reducing a saccharide and the effect of producing trisaccharides were examined when the cyclodextrin-producing enzyme was inactivated after the treatment with the cyclodextrin-producing enzyme and then treated with the maltotriose-producing enzyme.

### (1) Experimental method

Twelve point five grams of oat flour (PREMIUM OAT FLOUR, the carbohydrate content 67.7 g/100 g, Slow Food Kitchen) was added to 100 mL of water to suspend it, and cyclodextrin-producing enzyme (CGT-1: 6 U/g-raw material, CGT-2: 4 U/g-raw material) was added to the raw material. After the enzyme was added, the enzyme was allowed to act for 2 hours while stirring in a 60°C water bath, and then the enzyme was inactivated by treating at 95°C for 10 minutes. After cooling to 50°C, maltotriose-producing enzyme (6 U/g-raw material, 12 U/g-raw material) was added. After the enzyme was added, the enzyme was allowed to act for 2 hours while stirring in a 50°C water bath, and then the enzyme was inactivated by treating at 95°C for 10 minutes. One hundred ninety microliters of 1 mol/L trichloroacetic acid was added to 1.5 mL of the enzyme-inactivated oat solution, and the mixture was allowed to stand at room temperature for 10 minutes. After 10 minutes, the mixture was centrifuged (12,000 rpm, 10 minutes) to recover the supernatant. The recovered supernatant was suspended in an equal amount of diethyl ether added. The mixture was centrifuged (12,000 rpm, 10 minutes), the organic solvent layer was discarded, and the aqueous layer was recovered. This procedure was repeated twice. The recovered aqueous layer was added with an equal amount of 99.5% ethanol (for high performance liquid chromatography, manufactured by FUJIFILM Wako Pure Chemical Corp.), stirred, and filtered through a membrane filter for analysis. The analysis was performed using high performance liquid chromatography, and columns used were MCL GEL CK04S manufactured by Mitsubishi Chemical Corporation and TSK gel Amide-80 manufactured by Tosoh Corporation. The amount of each component produced was quantified from the peak area.

### (2) Result

The results are shown in Table 6 below.

**[Table 6]**

| | Unit | Example | | | |
|---|---|---|---|---|---|
| | | 50 | 51 | 52 | 53 |
| Amount of CGT-1 added | U/g-raw material | 6 | 6 | 0 | 0 |
| Amount of CGT-2 added | U/g-raw material | 0 | 0 | 4 | 4 |
| Amount of maltotriose-producing enzyme added | U/g-raw material | 6 | 12 | 6 | 12 |
| Total saccharide (monosaccharide + disaccharide) | mg/mL | 2.4 | 3.5 | 2.3 | 3.8 |
| Trisaccharide | mg/mL | 9.0 | 13.6 | 10.3 | 11.0 |
| α-cyclodextrin | mg/mL | 4.7 | 4.8 | 7.0 | 7.0 |

### (3) Observation

As shown in Table 6, by treating with the maltotriose-producing enzyme after inactivation after the cyclodextrin-producing enzyme treatment, it was possible to increase the amount of trisaccharides produced and reduce the amount of saccharides (monosaccharides + disaccharides) produced at the same time. Furthermore, the production of α-cyclodextrin could also be confirmed. From these results, it is presumed that the oat milk obtained this time has a sweet taste and a reduced grain odor, despite having a low content of saccharides (monosaccharides + disaccharides).

## Claims

1. An enzymatic agent for reducing a saccharide in a plant-base food or beverage, comprising a cyclodextrin-producing enzyme.

2. The enzymatic agent for reducing a saccharide in a plant-base food or beverage according to claim 1, comprising a maltotriose-producing enzyme.

3. A plant-base food or beverage , in which the enzymatic agent for reducing a saccharide as described in claim 1 or 2 is used.

4. The plant-base food or beverage according to claim 3, wherein the plant-base food or beverage is oat milk.

5. A method for manufacturing a plant-base food or beverage, comprising a step of allowing a cyclodextrin-producing enzyme to act in a liquefaction process of a plant-base raw material.

6. The method for manufacturing a plant-base food or beverage according to claim 5, comprising a step of allowing a maltotriose-producing enzyme to act on a plant-base raw material.

7. A method for reducing a saccharide in a plant-base food or beverage, comprising a step of allowing a cyclodextrin-producing enzyme to act on a plant-base raw material.

8. The method for reducing a saccharide in a plant-base food or beverage according to claim 7, comprising a step of allowing a maltotriose-producing enzyme to act on a plant-base raw material.
